(19) Europäisches Patentamt
European Patent Office
Office européen des brevets

(11) Veröffentlichungsnummer: **0 154 242**
**A2**

(12) # EUROPÄISCHE PATENTANMELDUNG

(21) Anmeldenummer: **85101792.1**

(22) Anmeldetag: **19.02.85**

(51) Int. Cl.[4]: **C 07 C 157/12**
C 07 C 103/90, C 07 D 233/42
C 07 D 213/64, A 01 N 47/34
A 01 N 43/50

(30) Priorität: **01.03.84 DE 3407493**

(43) Veröffentlichungstag der Anmeldung:
**11.09.85 Patentblatt 85/37**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Kraatz, Udo, Dr.**
**Andreasstrasse 22a**
**D-5090 Leverkusen 1(DE)**

(72) Erfinder: **Führer, Wolfgang, Dr.**
**Wehrstrasse 28**
**D-5202 Hennef 1(DE)**

(72) Erfinder: **Eue, Ludwig, Dr.**
**Paul-Klee-Strasse 36**
**D-5090 Leverkusen 1(DE)**

(72) Erfinder: **Schmidt, Robert R, Dr.**
**Im Waldwinkel 110**
**D-5060 Bergisch-Gladbach 2(DE)**

(72) Erfinder: **Santel, Hans-Joachim, Dr.**
**Gerstenkamp 19**
**D-5000 Köln 80(DE)**

(54) **Substituierte Phenoxypropionsäure-Derivate.**

(57) Neue substituierte Phenoxypropionsäure-Derivate der Formel

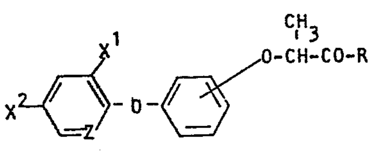 (I)

in welcher

$X^1$ für Wasserstoff oder Halogen steht,
$X^2$ für Trifluormethyl oder Halogen steht,
Z für Stickstoff oder den Rest CX steht, worin
X für Wasserstoff oder Halogen steht und
R für den Rest der Formel

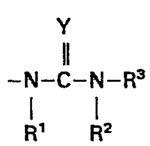

steht, worin
Y für Sauerstoff oder Schwefel steht,
$R^1$ für Wasserstoff, Alkyl mit 1 bis 6 Kohlenstoffatomen oder Aryl steht,
$R^2$ für Wasserstoff, Alkyl mit 1 bis 6 Kohlenstoffatomen, Alkenyl mit 3 bis 8 Kohlenstoffatomen oder gegebenenfalls substituiertes Aryl steht, oder
$R^1$ und $R^2$ gemeinsam für eine gegebenenfalls substituierte Alkylenkette mit 2 bis 4 Kohlenstoffatomen stehen, und
$R^3$ für Wasserstoff, Alkyl mit 1 bis 6 Kohlenstoffatomen oder für gegebenenfalls substituiertes Aryl steht, oder
R für den Rest der Formel

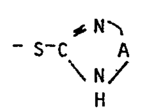

steht, worin
A für eine gegebenenfalls substituierte Alkylenkette mit 2 bis 4 Kohlenstoffatomen steht, oder
R für den Rest der Formel 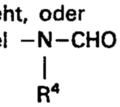

steht, worin
$R^4$ für Wasserstoff oder Alkyl mit 1 bis 6 Kohlenstoffatomen steht,
mehrere Verfahren zur Herstellung der neuen Stoffe und deren Verwendung als Herbizide.

EP 0 154 242 A2

Croydon Printing Company Ltd.

0154242

BAYER AKTIENGESELLSCHAFT
5090 Leverkusen, Bayerwerk
Konzernverwaltung RP
Patentabteilung
Dü/Ke-c
Ib

Substituierte Phenoxypropionsäure-Derivate

Die Erfindung betrifft neue substituierte Phenoxypropionsäure-Derivate, mehrere Verfahren zu ihrer Herstellung und ihre Verwendung als Herbizide.

Es ist bereits bekannt geworden, daß zahlreiche Phenoxypropionsäure-Derivate herbizide Eigenschaften besitzen (vgl. DE-OS 22 23 894). So kann zum Beispiel der 2-[4-(2,4-Dichlorphenoxy)-phenoxy]-propionsäure-methylester zur Unkrautbekämpfung eingesetzt werden. Die Wirkung dieses Stoffes ist jedoch insbesondere beim Einsatz niedriger Aufwandmengen nicht immer ausreichend.

Es wurden nun neue substituierte Phenoxypropionsäure-Derivate der Formel

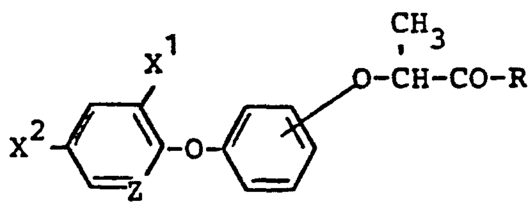 (I)

in welcher

$X^1$ für Wasserstoff oder Halogen steht,

$X^2$ für Trifluormethyl oder Halogen steht,

Z für Stickstoff oder den Rest CX steht, worin X für Wasserstoff oder Halogen steht und

R für den Rest der Formel

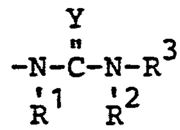

steht, worin

Y für Sauerstoff oder Schwefel steht,

$R^1$ für Wasserstoff , Alkyl mit 1 bis 6 Kohlenstoffatomen oder Aryl steht,

$R^2$ für Wasserstoff, Alkyl mit 1 bis 6 Kohlenstoffatomen, Alkenyl mit 3 bis 8 Kohlenstoffatomen oder gegebenenfalls substituiertes Aryl steht,

oder

$R^1$ und $R^2$ gemeinsam für eine gegebenenfalls substituierte Alkylenkette mit 2 bis 4 Kohlenstoffatomen stehen,

und

$R^3$ für Wasserstoff, Alkyl mit 1 bis 6 Kohlenstoffatomen oder für gegebenenfalls substituiertes Aryl steht,

oder

R für den Rest der Formel

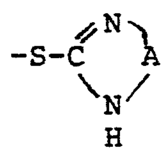

steht, worin

A für eine gegebenenfalls substituierte Alkylenkette mit 2 bis 4 Kohlenstoffatomen steht,

oder

R für den Rest der Formel $-\underset{R^4}{\underset{|}{N}}-CHO$

steht, worin

$R^4$ für Wasserstoff oder Alkyl mit 1 bis 6 Kohlenstoffatomen steht,

gefunden.

Die substituierten Phenoxypropionsäure-Derivate der Formel (I) enthalten mindestens ein asymmetrisch substituiertes Kohlenstoffatom und können deshalb in verschiedenen enantiomeren Formen vorliegen. Die Erfindung betrifft sowohl die möglichen einzelnen Isomeren als auch Gemische dieser Isomeren.

Weiterhin wurde gefunden, daß man neue substituierte Phenoxypropionsäure-Derivate der Formel (I) erhält, wenn man

a) Phenoxypropionsäurechloride der Formel

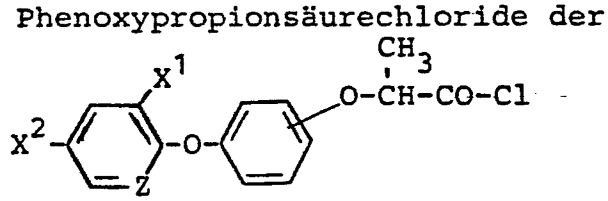

(II)

in welcher

$X^1$, $X^2$ und Z die oben angegebene Bedeutung haben,

mit Verbindungen der Formel

$$H - R \qquad (III)$$

in welcher
R die oben angegebene Bedeutung hat,
in Gegenwart eines Verdünnungsmittels und gegebenenfalls in Gegenwart eines Säurebindemittels umsetzt,

oder

b) Phenoxypropionsäure-Derivate der Formel

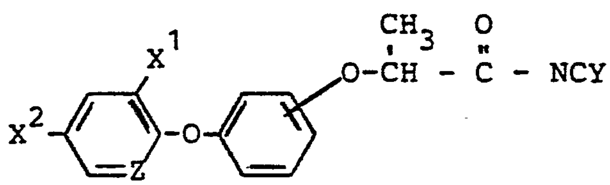

(IV)

in welcher
$X^1$, $X^2$, Y und Z die oben angegebene Bedeutung haben,

mit Aminen der Formel

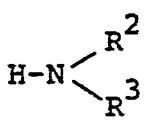

(V)

in welcher
$R^2$ und $R^3$ die oben angegebene Bedeutung haben,

gegebenenfalls in Gegenwart eines Verdünnungsmittels umsetzt.

Schließlich wurde gefunden, daß sich die neuen substituierten Phenoxypropionsäure-Derivate der Formel (I) durch eine hervorragende herbizide Wirksamkeit auszeichnen.

Überraschenderweise besitzen die erfindungsgemäßen substituierten Phenoxypropionsäure-Derivate der Formel (I) wesentlich bessere herbizide Eigenschaften als der aus dem Stand der Technik bekannte 2-[4-(2,4-Dichlorphenoxy)-phenoxy]-propionsäure-methylester, welcher ein hochwirksamer Wirkstoff gleicher Wirkungsart ist.

Die erfindungsgemäßen substituierten Phenoxypropionsäure-Derivate sind durch die Formel (I) allgemein definiert. In dieser Formel steht

$X^1$ vorzugsweise für Wasserstoff, Fluor, Chlor und Brom,

$X^2$ vorzugsweise für Trifluormethyl, Fluor, Chlor und Brom,

Z für Stickstoff oder den Rest CX, worin
X vorzugsweise für Wasserstoff, Fluor, Chlor oder Brom steht, und

R für den Rest der Formel $-\underset{R^1}{N}-\overset{\overset{Y}{\|}}{C}-\underset{R^2}{N}-R^3$,

worin
Y für Sauerstoff oder Schwefel steht,
$R^1$ vorzugsweise für Wasserstoff oder geradkettiges, verzweigtes Alkyl mit 1 bis 4 Kohlenstoffatomen oder Phenyl steht,

$R^2$ vorzugsweise für Wasserstoff, geradkettiges oder verzweigtes Alkyl mit 1 bis 4 Kohlenstoffatomen, Alkenyl mit 3 bis 6 Kohlenstoffatomen oder für gegebenenfalls durch Halogen und/oder Alkyl mit 1 bis 4 Kohlenstoffatomen substituiertes Phenyl steht, oder

$R^1$ und $R^2$ gemeinsam vorzugsweise für eine gegebenenfalls durch Halogen und/oder Alkyl mit 1 bis 4 Kohlenstoffatomen substituierte Alkylenkette mit 2 oder 3 Kohlenstoffatomen stehen, und

$R^3$ vorzugsweise für Wasserstoff, geradkettiges oder verzweigtes Alkyl mit 1 bis 4 Kohlenstoffatomen oder für gegebenenfalls durch Halogen und/oder Alkyl mit 1 bis 4 Kohlenstoffatomen substituiertes Phenyl steht,

oder

R steht für den Rest der Formel 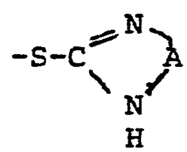

worin

A vorzugsweise für eine gegebenenfalls durch Alkyl mit 1 oder 2 Kohlenstoffatomen und/oder Halogen substituierte Alkylenkette mit 2 bis 4 Kohlenstoffatomen steht, oder

R steht für den Rest der Formel $-\overset{R^4}{\overset{|}{N}}-CHO$,

worin

$R^4$ vorzugsweise für Wasserstoff oder geradkettiges oder verzweigtes Alkyl mit 1 bis 4 Kohlenstoffatomen steht.

Besonders bevorzugt sind diejenigen substituierten Phenoxypropionsäure-Derivate der Formel (I) in denen

$X^1$ für Wasserstoff, Fluor oder Chlor steht,

$X^2$ für Trifluormethyl, Fluor oder Chlor steht,

Z für Stickstoff oder den Rest CX steht, worin X für Wasserstoff, Fluor oder Chlor steht, und

R für den Rest der Formel $$-\underset{R^1}{\underset{|}{N}}-\overset{Y}{\overset{\|}{C}}-\underset{R^2}{\underset{|}{N}}-R^3$$

steht, worin

Y für Sauerstoff oder Schwefel steht,

$R^1$ für Wasserstoff, Methyl, Ethyl, n-Propyl, iso-Propyl, n-Butyl, sek.-Butyl, iso-Butyl, tert.-Butyl oder Phenyl steht,

$R^2$ für Wasserstoff, Methyl, Ethyl, n-Propyl, iso-Propyl, n-Butyl, sek.-Butyl, iso-Butyl, tert.-Butyl, geradkettiges oder verzweigtes Alkenyl mit 3 bis 5 Kohlenstoffatomen oder für gegebenenfalls durch Fluor, Chlor, Methyl, Ethyl, n-Propyl und/oder iso-Propyl substituiertes Phenyl steht,

oder

$R^1$ und $R^2$ gemeinsam für eine gegebenenfalls durch Chlor, Methyl und/oder Ethyl substituierte Alkylenkette mit 2 oder 3 Kohlenstoffatomen stehen, und

$R^3$ für Wasserstoff, Methyl, Ethyl, n-Propyl, iso-Propyl, n-Butyl, sek.-Butyl, iso-Butyl, tert.-Butyl oder für gegebenenfalls durch Fluor,

Chlor, Methyl, Ethyl, n-Propyl und/oder iso-Propyl substituiertes Phenyl steht,

oder

R für den Rest der Formel 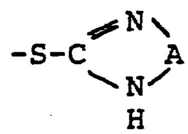 steht,

worin

A für eine gegebenenfalls durch Chlor, Methyl und/oder Ethyl substituierte Alkylenkette mit 2 oder 3 Kohlenstoffatomen steht,

oder

R für den Rest der Formel 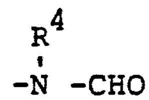 steht, worin

$R^4$ für Wasserstoff, Methyl, Ethyl, n-Propyl, iso-Propyl, n-Butyl, sek.-Butyl, iso-Butyl oder tert.-Butyl steht.

Eine besonders bevorzugte Gruppe von substituierten Phenoxypropionsäure-Derivaten sind auch die Verbindungen der Formel

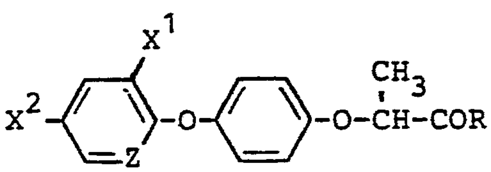

(Ia)

in welcher

$X^1$, $X^2$, Z und R diejenigen Bedeutungen haben, die oben als bevorzugt genannt wurden.

Eine weitere Gruppe besonders bevorzugter erfindungsgemäßer Stoffe sind die Verbindungen der Formel

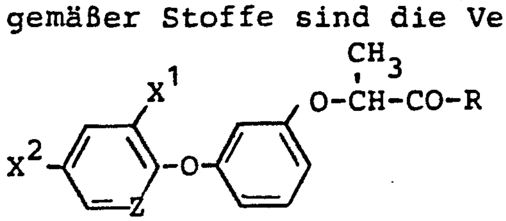

(Ib)

in welcher

$X^1$, $X^2$, Z und R diejenigen Bedeutungen haben, die oben als bevorzugt genannt wurden.

Eine weitere Gruppe besonders bevorzugter erfindungsgemäßer Verbindungen sind schließlich diejenigen Verbindungen der Formeln (Ia) und (Ib), in denen das asymmetrisch substituierte Kohlenstoffatom der Propionsäure-Einheit die R-Konfiguration aufweist. Die betreffenden Stoffe lassen sich durch die Formeln

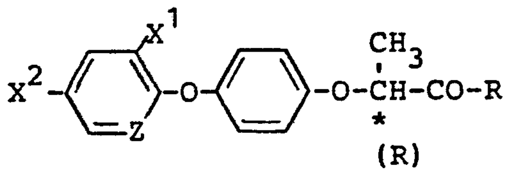

(Ia')

und

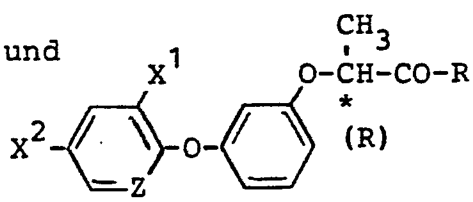

(Ib')

charakterisieren. In diesen Verbindungen ist das asymmetrisch substituierte Kohlenstoffatom jeweils durch ein (*) gekennzeichnet.

Verwendet man 2-[4-(4-Trifluormethylphenoxy)-phenoxy]-propionsäurechlorid und N-Methyl-thioharnstoff als Ausgangssubstanzen, so kann der Verlauf des erfindungsgemäßen Verfahrens (a) durch das folgende Formelschema wiedergegeben werden.

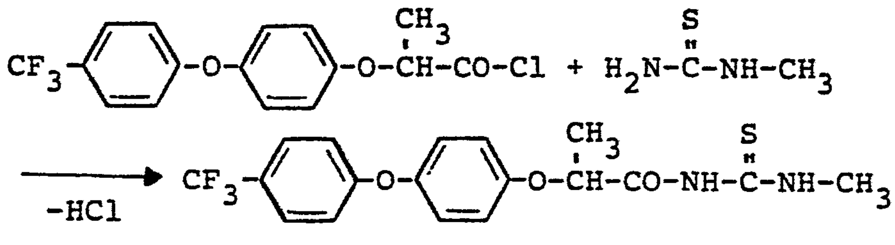


Verwendet man 2-[4-(2-Chlor-4-trifluormethyl-phenoxy)-phenoxy]-propionsäureisothiocyanat und n-Butylamin als Ausgangsstoffe, so kann der Verlauf des erfindungsgemäßen Verfahrens (b) durch das folgende Formelschema wiedergegeben werden:

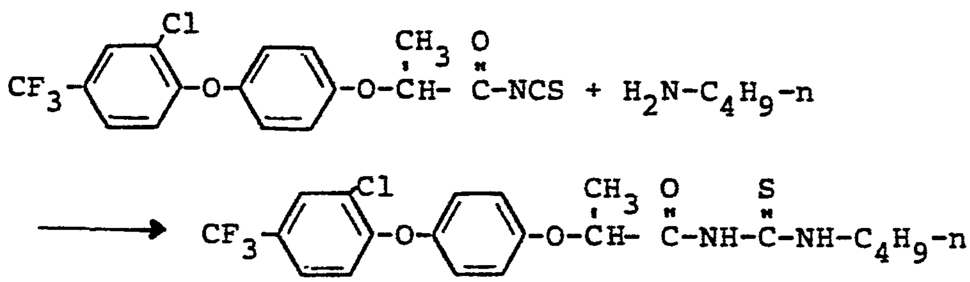

Die bei dem erfindungsgemäßen Verfahren (a) als Ausgangsstoffe benötigten Phenoxypropionsäurechloride sind durch die Formel (II) definiert. In dieser Formel haben $X^1$, $X^2$ und Z vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für Verbindungen der Formel (II) seien im einzelnen genannt:

2-[4-(4-Trifluormethyl-phenoxy)-phenoxy], 2-[4-(2,4-Dichlorphenoxy)-phenoxy]-, 2-[4-(2,6-Dichlor-4-trifluormethylphenoxy)-phenoxy]-, 2-[4-(2-Chlor-4-trifluormethyl-phenoxy)-, 2-[4-(3,5-Dichlor-pyridyl-2-oxy)-phenoxy]-, 2-[4-(3-Chlor-5-trifluormethyl-pyridyl-2-oxy)-phenoxy]- und 2-[4-(5-Trifluormethyl-pyridyl-2-oxy)-phenoxy]-propionsäurechlorid sowie 2-[3-(4-Trifluormethyl-phenoxy)-phenoxy]-, 2-[3-(2,4-Dichlorphenoxy)-phenoxy]-, 2-[3-(2,6-Dichlor-4-trifluormethyl-phenoxy)-phenoxy]-, 2-[3-(2-Chlor-4-trifluormethyl-phenoxy)-phenoxy]-, 2-[3-(3,5-Dichlor-pyridyl-2-oxy)-phenoxy]-, 2-[3-(3-Chlor-5-trifluormethyl-pyridyl-2-oxy)-phenoxy]- und 2-[3-(5-Trifluormethyl-pyridyl-2-oxy)-phenoxy]-propionsäurechlorid.

Die Phenoxypropionsäurechloride der Formel (II) sind bekannt oder lassen sich nach bekannten Verfahren in einfacher Weise herstellen (vgl. Deutsche Offenlegungsschriften 22 23 894, 24 33 067, 25 46 251, 26 46 124, 26 28 384 und 28 02 818 sowie Europäische Offenlegungsschriften 0 000 483 und 0 001 473).

Man erhält die Phenoxypropionsäurechloride der Formel (II) zum Beispiel dadurch, daß man Phenol-Derivate der Formel

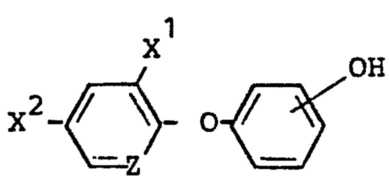

(VI)

in welcher

$X^1$, $X^2$ und Z die oben angegebene Bedeutung haben,

mit Propionsäure-Derivaten der Formel

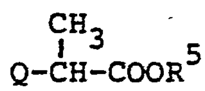

(VII)

in welcher

$R^5$ für Methyl oder Ethyl steht und
Q für Chlor, Brom, Mesylat oder Tosylat steht,

gegebenenfalls in Gegenwart eines Säureakzeptors, wie z.B. Kaliumcarbonat und gegebenenfalls in Gegenwart eines Verdünnungsmittels, wie zum Beispiel Acetonitril, bei Temperaturen zwischen 0°C und 120°C umsetzt und die dabei entstehenden Ester der Formel

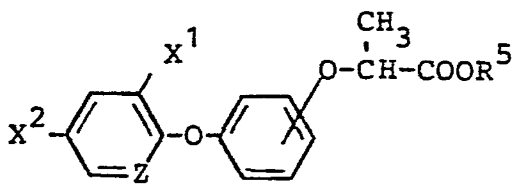

(VIII)

in welcher

$X^1$, $X^2$, $R^5$ und Z die oben angegebene Bedeutung haben,

mit starken Basen, wie zum Beispiel Natriumhydroxid, in Gegenwart eines Verdünnungsmittels, wie zum Beispiel Methanol, Ethanol, Benzol, Toluol oder Xylol, gegebenenfalls im Gemisch mit Wasser bei Temperaturen zwischen 20°C und 140°C verseift, dann mit einer Säure, wie zum Beispiel Salzsäure ansäuert und die dabei entstehenden Säuren der Formel

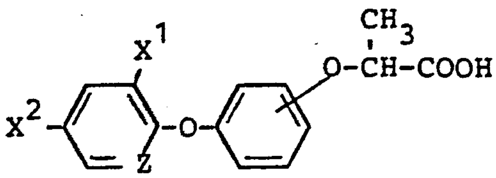

(IX)

in welcher

$X^1$, $X^2$ und Z die oben angegebene Bedeutung haben,

mit Chlorierungsmitteln, wie zum Beispiel Thionylchlorid, gegebenenfalls in Gegenwart eines inerten Verdünnungsmittels, wie zum Beispiel Tetrachlormethan, und gegebenenfalls in Gegenwart eines Katalysators, wie zum Beispiel Dimethylformamid, bei Temperaturen zwischen 20°C und 100°C umsetzt.

Zur Herstellung von optisch aktiven Phenoxypropionsäurechloriden der Formel

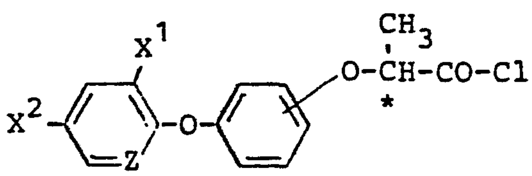

(II*)

in welcher

$X^1$, $X^2$ und Z die oben angegebene Bedeutung haben,

ist es erforderlich, bei dem oben beschriebenen Verfahren optisch aktive Propionsäure-Derivate der Formel

$$Q-\overset{CH_3}{\overset{|}{C}}H-COOR^5 \quad (VII^*)$$
$$\ \ \ \ *$$

in welcher

Q und $R^5$ die oben angegebene Bedeutung haben,

einzusetzen. Bei dieser Umsetzung findet am asymmetrisch substituierten Kohlenstoffatom der Propionsäure-Einheit eine Walden'sche Umkehr statt. Dieses hat zur Folge, daß durch Umsetzung eines Phenols der Formel (VI) mit einem S-Enantiomeren eines Propionsäure-Derivates der Formel (VII) das entsprechende R-Enantiomere eines Esters der Formel (VIII) und schließlich auch das R-Enantiomere eines Phenoxypropionsäurechlorids der Formel (II) entsteht. Andererseits bildet sich durch Umsetzung eines Phenols der Formel (VI) mit einem R-Enantiomeren eines Propionsäure-Derivates der Formel (VII) zunächst das entsprechende S-Enantiomere eines Esters der Formel (VIII) und dann das entsprechende S-Enantiomere eines Phenoxypropionsäurechlorids der Formel (II).

Die bei dem erfindungsgemäßen Verfahren (a) weiterhin als Ausgangsstoffe benötigten Verbindungen sind durch die Formel (III) definiert. In dieser Formel hat R vorzugsweise diejenigen Bedeutungen, die bereits im

Zusammenhang mit der Beschreibung der erfindungsgemäßen Stoffe der Formel (I) vorzugsweise für diesen Rest genannt wurden.

Die Verbindungen der Formel (III) sind bekannt.

Das erfindungsgemäße Verfahren (a) wird unter Verwendung von Verdünnungsmitteln durchgeführt. Als Verdünnungsmittel kommen hierbei praktisch alle inerten, aprotischen organischen Solventien infrage. Vorzugsweise verwendbar sind aliphatische und aromatische, gegebenenfalls halogenierte Kohlenwasserstoffe, wie Pentan, Hexan, Heptan, Cyclohexan, Benzol, Toluol, Xylol, Methylenchlorid, Chloroform, Tetrachlorkohlenstoff, Chlorbenzol und Dichlorbenzol, Ether, wie Diethylether, Diisopropylether, 1,2-Dimethoxy-ethan, Tetrahydrofuran und Dioxan, Ester, wie Essigsäuremethylester und -ethylester, Nitrile, wie Acetonitril und Propionitril, Amide, wie Dimethylformamid und Dimethylacetamid, sowie Dimethylsulfoxid und Sulfolan.

Die Umsetzung nach dem erfindungsgemäßen Verfahren (a) kann sowohl in Anwesenheit als auch in Abwesenheit von Säurebindemitteln vorgenommen werden. Arbeitet man in Gegenwart von Säurebindemitteln, so kommen als derartige Säureakzeptoren praktisch alle üblichen Säurebindemittel in Betracht.

Besonders bevorzugt verwendbar sind Alkalimetall- und Erdalkalimetallhydroxide, wie z.B. Natrium-, Kalium-und Calcium-hydroxid, Alkalimetall- und Erdalkalimetallcarbonate bzw. -hydrogen-carbonate, wie z.B. Natriumcarbonat und -hydrogencarbonat, Kaliumcarbonat und Calciumcarbonat, sowie aliphatische, aromatische und heterocyclische Amine, wie z.B. Trimethyl-, Triethyl-, Tripropyl- und Tributyl-amin, N,N-Dimethyl-benzylamin, Pyridin, 2-Methylpyridin, 2,4,6-Trimethyl-pyridin, 2-Methyl-5-ethyl-pyridin, Diazabicyclooctan (DABCO), Diazabicyclononen (DBN) und Diazabicycloundecen (DBU).

Die Reaktionstemperaturen können bei dem Verfahren (a) innerhalb eines größeren Bereiches variiert werden. Im allgemeinen arbeitet man bei Temperaturen zwischen -50°C und +200°C, vorzugsweise zwischen -20°C und +150°C.

Das Verfahren (a) wird im allgemeinen bei Normaldruck durchgeführt.

Zur Durchführung des erfindungsgemäßen Verfahrens (a) setzt man pro Mol an Ausgangsverbindung der Formel (II) im allgemeinen zwischen 0,8 und 4,0 Mol, vorzugsweise zwischen 0,9 und 3,0 Mol Ausgangsverbindung der Formel (III) ein. Arbeitet man in Gegenwart von Säurebindemitteln, so werden diese in äquivalenter Menge oder auch in einem Überschuß, bezogen auf Phenoxypropionsäurechlorid der Formel (II), eingesetzt. Die Durchführung der Umsetzung erfolgt beim Arbeiten ohne Säurebindemittel im allgemeinen in der Weise, daß man die Komponenten zusammengibt und dann bei erhöhter Temperatur

reagieren läßt. Arbeitet man in Gegenwart eines Säurebindemittels, so werden die Komponenten gegebenenfalls unter Kühlung zusammengegeben, und das Reaktionsgemisch wird danach gegebenenfalls bei erhöhter Temperatur gerührt. Die anschließende Aufarbeitung erfolgt nach üblichen Methoden. Im allgemeinen geht man so vor, daß man den festen Rückstand abfiltriert, mit einem organischen Lösungsmittel extrahiert und die vereinigten organischen Phasen wäscht und einengt. Es ist jedoch auch möglich, das Reaktionsgemisch nach beendeter Umsetzung mit Wasser zu verdünnen, das entstehende Gemisch mit einem mit Wasser wenig mischbaren organischen Lösungsmittel zu extrahieren, die vereinigten organischen Phasen zu waschen und dann einzuengen. Die dabei anfallenden Produkte können durch übliche Maßnahmen, wie Umkristallisation oder auf chromatographischem Wege von eventuell noch enthaltenen Verunreinigungen befreit werden.

Die bei dem erfindungsgemäßen Verfahren (b) als Ausgangsstoffe benötigten Phenoxypropionsäure-Derivate sind durch die Formel (IV) definiert. In dieser Formel haben $X^1$, $X^2$, Y und Z vorzugsweise diejenigen Bedeutungen, die bereits im Zusammenhang mit der Beschreibung der erfindungsgemäßen Stoffe der Formel (I) vorzugsweise für diese Reste genannt wurden.

Die Verbindungen der Formel (IV) sind bekannt oder lassen sich nach im Prinzip bekannten Verfahren in einfacher Weise herstellen. So erhält man Phenoxypropionsäure-Derivate der Formel (IV) zum Beispiel dadurch, daß man Phenoxypropionsäurechloride der Formel

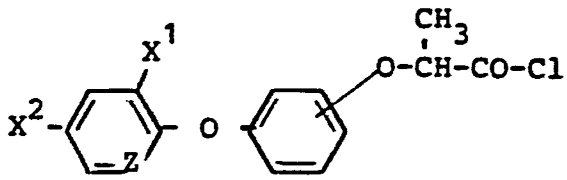

(II)

in welcher

$X^1$, $X^2$ und Z die oben angegebene Bedeutung haben,

entweder mit Isocyanaten oder Isothiocyanaten der Formel

YCN-M (X)

in welcher

Y die oben angegebene Bedeutung hat und
M für Natrium oder Kalium steht,

oder mit Trimethylsilyl-isothiocyanat der Formel

$SCN-Si(CH_3)_3$ (XI)

in Gegenwart eines inerten Verdünnungsmittels, wie Toluol oder Benzol, bei Temperaturen zwischen 50°C und 200°C umsetzt.

Zur Herstellung von optisch aktiven Verbindungen der Formel (IV) werden die entsprechenden optisch aktiven

Phenoxypropionsäurechloride der Formel (II) eingesetzt.

Die bei der Durchführung des erfindungsgemäßen Verfahrens (b) als Reaktionskomponenten benötigten Verbindungen sind durch die Formel (V) definiert. In dieser Formel haben $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden. Die Amine der Formel (V) sind bekannt.

Als Verdünnungsmittel können bei der Durchführung des erfindungsgemäßen Verfahrens (b) alle inerten, aprotischen organischen Lösungsmittel verwendet werden. Vorzugsweise in Betracht kommen alle diejenigen Solventien, die bereits im Zusammenhang mit der Beschreibung des erfindungsgemäßen Verfahrens (a) vorzugsweise genannt wurden.

Die Reaktionstemperaturen können auch bei der Durchführung des erfindungsgemäßen Verfahrens (b) innerhalb eines größeren Bereiches variiert werden. Im allgemeinen arbeitet man bei Temperaturen zwischen 0°C und 100°C, vorzugsweise zwischen 10°C und 80°C.

Das erfindungsgemäße Verfahren wird im allgemeinen unter Atmosphärendruck durchgeführt.

Bei der Durchführung des erfindungsgemäßen Verfahrens (b) setzt man die Komponenten der Formeln (IV) und (V)

im allgemeinen in äquivalenten Mengen ein. Es ist jedoch auch möglich, eine der beiden Komponenten im einem Überschuß einzusetzen. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich insbesondere zur Gräserbekämpfung in dikotylen Kulturpflanzen wie Baumwolle, Raps, Sojabohnen, Zuckerrüben sowie in Getreide, wie zum Beispiel Weizen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 4-Amino-6-(1,1 -dimethylethyl)-3-ethylthio-1,2,4-triazin-5(4H)on, 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden. Die Anwendung erfolgt insbesondere nach dem Auflaufen der Pflanzen.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

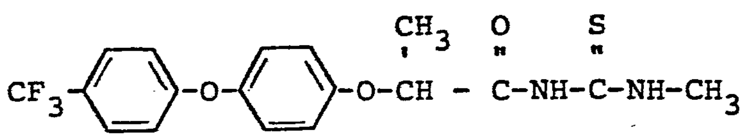

Ein Gemisch aus 173 g (0,5 Mol) 2-/4-(4-Trifluormethyl-phenoxy)-phenoxy7-propionsäurechlorid und 126 g (1,4 Mol) N-Methyl-thioharnstoff in 150 ml Toluol wurde unter Rühren 4 Stunden unter Rückfluß erhitzt. Nach dem Abkühlen wurde vom ausgefallenen Niederschlag abfiltriert. Der Rückstand wurde mit Methylenchlorid ausgekocht, und die so erhaltene Methylenchlorid-Lösung wurde nach dem Filtrieren mit der Toluolphase vereinigt. Die organische Phase wurde unter vermindertem Druck eingeengt, und der verbleibende Rückstand wurde erneut in Methylenchlorid gelöst. Man wusch die organische Phase zweimal mit Wasser und engte dann unter vermindertem Druck ein. Der verbleibende Rückstand wurde aus einem Gemisch von Cyclohexan und Isopropanol (1:1) umkristallisiert. Man erhielt auf diese Weise 180 g (90 % der Theorie) an 1-(2-/4-(4-Trifluormethyl-phenoxy)-phenoxy7-propionyl)-3-methyl-thioharnstoff in Form einer Festsubstanz vom Schmelzpunkt 100°C.

Beispiel 2

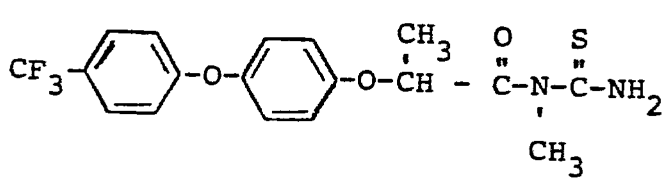

In ein Gemisch aus 9 g (0,1 Mol) N-Methylthioharnstoff und 12 g (0,15 Mol) Pyridin in 50 ml Dimethylformamid wurden bei 20°C unter Rühren und unter gelegentlicher Kühlung 35 g (0,1 Mol) 2-[4-(4-Trifluormethyl-phenoxy)-phenoxy]-propionsäurechlorid eingetropft. Man ließ 1 Stunde bei 20°C nachrühren und arbeitete dann auf, indem man das Reaktionsgemisch in Wasser goß und mit Methylenchlorid extrahierte. Die organische Phase wurde mit verdünnter wäßriger Salzsäure gewaschen und dann unter vermindertem Druck eingeengt. Das anfallende Produktgemisch wurde mit Hilfe einer Säule chromatographisch getrennt (Laufmittel: Chloroform/Essigester = 9:1).

Durch Eindampfen des zuerst aufgefangenen Eluates wurden 10,5 g (26,4 % der Theorie) an 1-(2-[4-(4-Trifluormethyl-phenoxy)-phenoxy]-3-methyl-thioharnstoff (= Verbindung gemäß Beispiel 1) erhalten. Fp. = 100°C.

Durch Eindampfen des danach auslaufenden Eluates wurden 12 g (30,2 % der Theorie) an 1-(2-[4-(4-Trifluormethyl-phenoxy)-phenoxy]-propionyl)-1-methyl-thioharnstoff erhalten. Fp. = 143°C.

Nach den in dem Beispielen 1 und 2 angegebenen Methoden wurden auch die in der folgenden Tabelle 1 formelmäßig aufgeführten Stoffe der Formel (I) hergestellt.

Tabelle 1

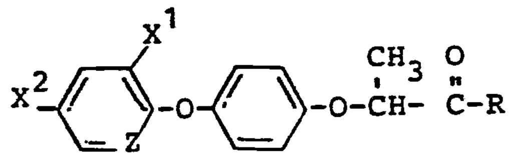

(Ia)

| Beispiel Nr. | $X^1$ | $X^2$ | Z | R | Schmelzpunkt (°C) |
|---|---|---|---|---|---|
| 3 | H | $CF_3$ | CH | 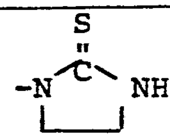 | 129 |
| 4 | H | $CF_3$ | CH | 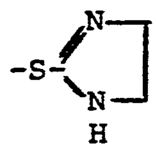 | 105-108 |
| 5 | H | $CF_3$ | CH | -NH-CHO | 141 |
| 6 | Cl | $CF_3$ | CCl | S<br>-NH-C-$NHCH_3$ | 85 |
| 7 | Cl | $CF_3$ | CH | S<br>-NH-C-$NH_2$ | 120 |
| 8 | Cl | $CF_3$ | CH | S<br>-NH-C-NH<br>$CH_2$-CH = $CH_2$ | Harz |
| 9 | Cl | $CF_3$ | CH | S<br>-NH-C-NH-$CH_3$ | Harz |

Tabelle 1 (Fortsetzung)

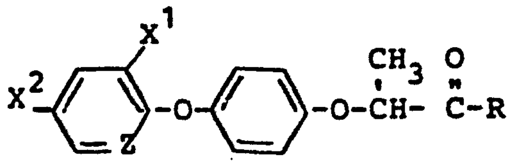

(Ia)

| Beispiel Nr. | $X^1$ | $X^2$ | Z | R | Schmelzpunkt (°C) |
|---|---|---|---|---|---|
| 1o | Cl | $CF_3$ | CCl | 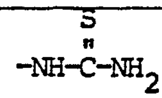 | 1o8 |
| 11 | Cl | $CF_3$ | CCl | 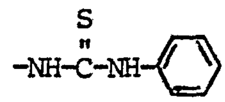 | 138 |
| 12 | Cl | $CF_3$ | CH | 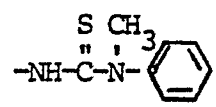 | Öl |
| 13 | H | $CF_3$ | CH | 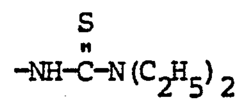 | 86-88 |
| 14 | H | $CF_3$ | CH | 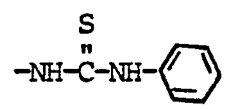 | 1o6 |
| 15 | Cl | $CF_3$ | CH | 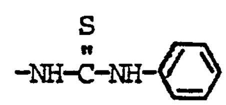 | 82 |
| 16 | Cl | $CF_3$ | CH | 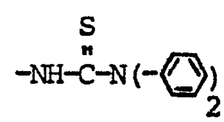 | 146 |
| 17 | Cl | $CF_3$ | CH | 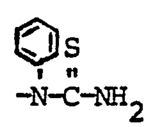 | 122 |
| 18 | H | $CF_3$ | CH | 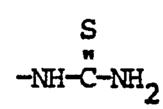 | 12o |

Nach den vorstehend angegebenen Methoden werden auch die in den folgenden Beispielen aufgeführten Stoffe erhalten.

Beispiel 19

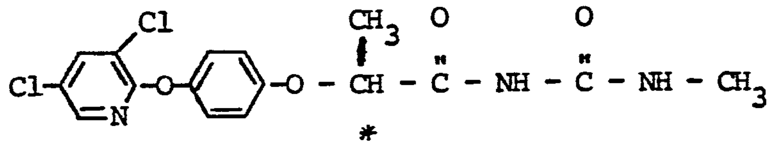

(R-Enantiomeres)

Schmelzpunkt: 1o2°C

Drehwert: $[\alpha]_D^{23} = + 75,8^\circ$ ( C = o,515 / $CHCl_3$)

Beispiel 2o

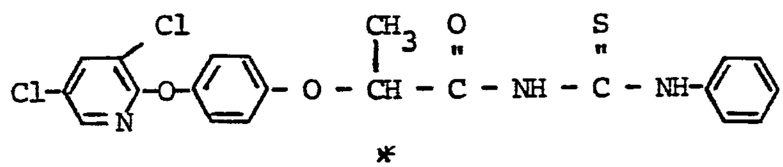

(R-Enantiomeres)

Schmelzpunkt: 83°C

Drehwert: $[\alpha]_D^{23} = + 49,5^\circ$ (C = 1,oo8 / $CHCl_3$)

Beispiel 21

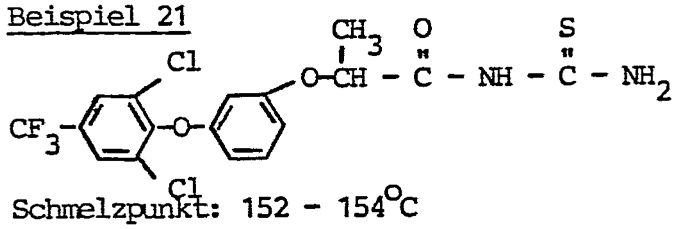

Schmelzpunkt: 152 - 154°C

Beispiel 22

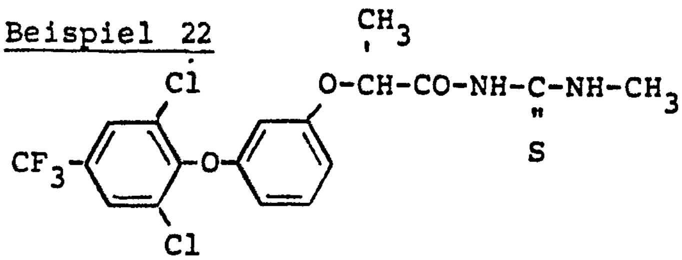

Die Verbindung der angegebenen Formel wurde nach der im Beispiel 1 beschriebenen Methode hergestellt.
Schmelzpunkt: 130°C

Verwendungsbeispiel

In dem nachstehend beschriebenen biologischen Test wurde die folgende Verbindung als Vergleichssubstanz eingesetzt:

(A) = 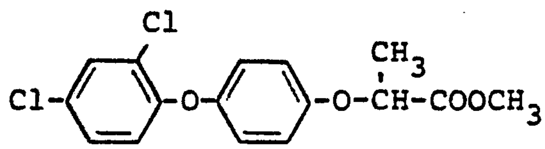

2-/4-(2,4-Dichlorphenoxy)-phenoxy/-propionsäure-methylester
(bekannt aus DE-OS 22 23 894)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit

Wasser auf die gewünschte Konzentration.

Mit der Wirkstoffzubereitung spritzt man Testpflanzen, welche eine Höhe von 5 - 15 cm haben, so daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigen die Wirkstoffe gemäß Beispielen (1), (2), (3) und (5 bei der Bekämpfung von Avena fatua, Digitaria, Echinochloa und Setaria in Zuckerrüben, Baumwolle und Soja eine bessere selektive herbizide Wirksamkeit als die Vergleichssubstanz (A).

Patentansprüche

1. Substituierte Phenoxypropionsäure-Derivate der Formel

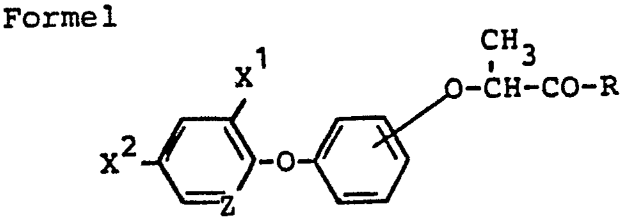

(I)

in welcher

$X^1$ für Wasserstoff oder Halogen steht,

$X^2$ für Trifluormethyl oder Halogen steht,

Z für Stickstoff oder den Rest CX steht, worin X für Wasserstoff oder Halogen steht und

R für den Rest der Formel

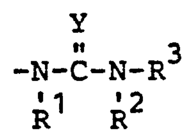

steht, worin
Y für Sauerstoff oder Schwefel steht,
$R^1$ für Wasserstoff , Alkyl mit 1 bis 6 Kohlenstoffatomen oder Aryl steht,
$R^2$ für Wasserstoff, Alkyl mit 1 bis 6 Kohlenstoffatomen, Alkenyl mit 3 bis 8 Kohlenstoffatomen oder gegebenenfalls substituiertes Aryl steht,
oder
$R^1$ und $R^2$ gemeinsam für eine gegebenenfalls substituierte Alkylenkette mit 2 bis 4 Kohlenstoffatomen stehen,
und

$R^3$ für Wasserstoff, Alkyl mit 1 bis 6 Kohlenstoffatomen oder für gegebenenfalls substituiertes Aryl steht,

oder

R für den Rest der Formel 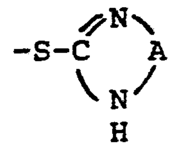

steht, worin

A für eine gegebenenfalls substituierte Alkylenkette mit 2 bis 4 Kohlenstoffatomen steht,

oder

R für den Rest der Formel 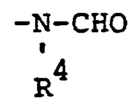

steht, worin

$R^4$ für Wasserstoff oder Alkyl mit 1 bis 6 Kohlenstoffatomen steht.

2. Substituierte Phenoxypropionsäure-Derivate der Formel (I), in denen

$X^1$ für Wasserstoff, Fluor, Chlor und Brom steht,

$X^2$ für Trifluormethyl, Fluor, Chlor und Brom steht,

Z für Stickstoff oder den Rest CX steht, worin
X für Wasserstoff, Fluor, Chlor oder Brom steht,

und

R für den Rest der Formel 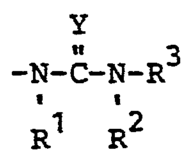 steht,

worin

Y für Sauerstoff oder Schwefel steht,

$R^1$ für Wasserstoff , geradkettiges oder verzweigtes Alkyl mit 1 bis 4 Kohlenstoffatomen oder Phenyl steht,

$R^2$ für Wasserstoff, geradkettiges oder verzweigtes Alkyl mit 1 bis 4 Kohlenstoffatomen, Alkenyl mit 3 bis 6 Kohlenstoffatomen oder für gegebenenfalls durch Halogen und/oder Alkyl mit 1 bis 4 Kohlenstoffatomen substituiertes Phenyl steht, oder

$R^1$ und $R^2$ gemeinsam für eine gegebenenfalls durch Halogen und/oder Alkyl mit 1 bis 4 Kohlenstoffatomen substituierte Alkylenkette mit 2 oder 3 Kohlenstoffatomen stehen, und

$R^3$ für Wasserstoff, geradkettiges oder verzweigtes Alkyl mit 1 bis 4 Kohlenstoffatomen oder für gegebenenfalls durch Halogen und/oder Alkyl mit 1 bis 4 Kohlenstoffatomen substituiertes Phenyl steht,

oder

R für den Rest der Formel 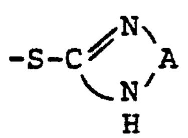 steht,

worin

A für eine gegebenenfalls durch Alkyl mit 1 oder 2 Kohlenstoffatomen und/oder Halogen substituierte Alkylenkette mit 2 bis 4 Kohlenstoffatomen steht,

oder

R für den Rest der Formel 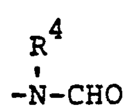 steht,

worin

$R^4$ für Wasserstoff oder geradkettiges oder verzweigtes Alkyl mit 1 bis 4 Kohlenstoffatomen steht.

3. Verfahren zur Herstellung von substituierten Phenoxypropionsäure-Derivaten der Formel

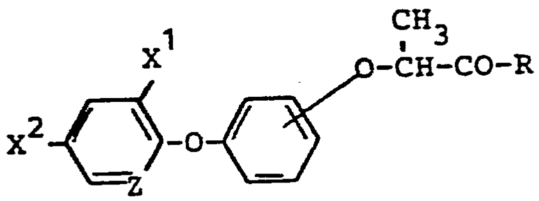

(I)

in welcher

$X^1$ für Wasserstoff oder Halogen steht,

$X^2$ für Trifluormethyl oder Halogen steht,

Z für Stickstoff oder den Rest CX steht, worin

X für Wasserstoff oder Halogen steht,

und

R für den Rest der Formel 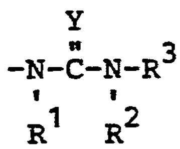

steht, worin

Y für Sauerstoff oder Schwefel steht,

$R^1$ für Wasserstoff, Alkyl mit 1 bis 6 Kohlenstoffatomen oder Aryl steht,

$R^2$ für Wasserstoff, Alkyl mit 1 bis 6 Kohlenstoffatomen, Alkenyl mit 3 bis 8 Kohlenstoffatomen oder gegebenenfalls substituiertes Aryl steht,

oder

$R^1$ und $R^2$ gemeinsam für eine gegebenenfalls substituierte Alkylenkette mit 2 bis 4 Kohlenstoffatomen stehen,

und

$R^3$ für Wasserstoff, Alkyl mit 1 bis 6 Kohlenstoffatomen oder für gegebenenfalls substituiertes Aryl steht,

oder

R für den Rest der Formel 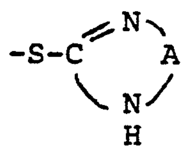

steht, worin

A für eine gegebenenfalls substituierte Alkylenkette mit 2 bis 4 Kohlenstoffatomen steht,

oder

R für den Rest der Formel 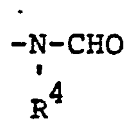

steht, worin

$R^4$ für Wasserstoff oder Alkyl mit 1 bis 6 Kohlenstoffatomen steht,

dadurch gekennzeichnet, daß man

a) Phenoxypropionsäurechloride der Formel

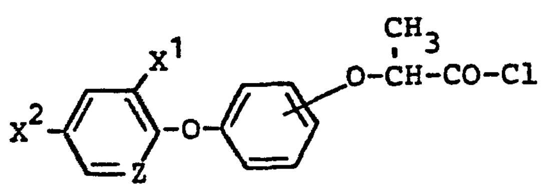

(II)

in welcher
$X^1$, $X^2$ und Z die oben angegebene Bedeutung haben,

mit Verbindungen der Formel

H - R (III)

in welcher
R die oben angegebene Bedeutung hat,

in Gegenwart eines Verdünnungsmittels und gegebenenfalls in Gegenwart eines Säurebindemittels umsetzt,
oder

b) Phenoxypropionsäure-Derivate der Formel

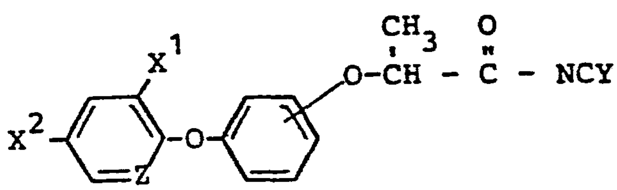

(IV)

in welcher
$X^1$, $X^2$, Y und Z die oben angegebene Bedeutung haben,

mit Aminen der Formel

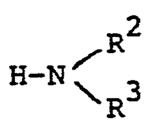

(V)

in welcher
$R^2$ und $R^3$ die oben angegebene Bedeutung haben,

gegebenenfalls in Gegenwart eines Verdünnungsmittels umsetzt.

4. Herbizide Mittel, gekennzeichnet durch einen Gehalt an mindestens einem substituierten Phenoxypropionsäure-Derivat der Formel (I).

5. Verfahren zur Bekämpfung von Unkräutern, dadurch gekennzeichnet, daß man substituierte Phenoxypropionsäure-Derivate der Formel (I) auf die Unkräuter und/oder deren Lebensraum ausbringt.

6. Verwendung von substituierten Phenoxypropionsäure-Derivaten der Formel (I) zur Bekämpfung von Unkräutern.

7. Verfahren zur Herstellung von herbiziden Mitteln, dadurch gekennzeichnet, daß man substituierte Phenoxypropionsäure-Derivate der Formel (I) mit Streckmitteln und/oder oberflächenaktiven Stoffen vermischt.

8. Substituiertes Phenoxypropionsäure-Derivat der Formel

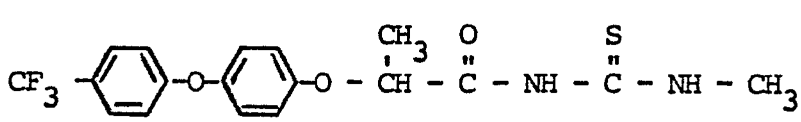

.

9. Substituiertes Phenoxypropionsäure-Derivat der Formel

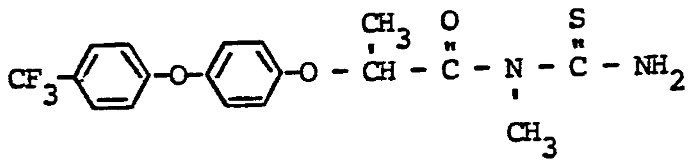

.

10. Substituiertes Phenoxypropionsäure-Derivat der Formel

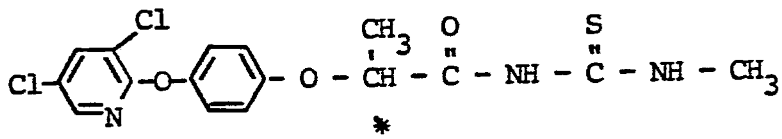

(R-Enantiomeres) .